# EUROPEAN PATENT SPECIFICATION

(11) **EP 1 827 397 B1**
(45) Date of publication and mention of the grant of the patent: **04.02.2009**
(21) Application number: 05800312.0
(22) Date of filing: 03.11.2005
(51) Int. Cl.: A61K 9/70

(54) **OVER DOSAGE INDICATING MEDICATED FILM STRIP**
MEDIKAMENTEN-FILMSTREIFEN ZUM HINWEIS VON ÜBERDOSIERUNG
BANDELETTE DE FILM MEDICAMENTEE INDICATRICE DE SURDOSAGE

(30) Priority: 15.11.2004 US 989212
(43) Date of publication of application: 05.09.2007
(73) Proprietor: McNeil-PPC, Inc., New Brunswick NJ 08933 (US)
(72) Inventor: LERNER, Keith S., Pfizer Global Research and Dev., Morris Plains, NJ 07950 (US)
(74) Representative: Mercer, Christopher Paul
(86) International application number: PCT/IB2005/003388
(87) International publication number: WO 2006/051406

(56) References cited:
- WO-A-97/48384
- WO-A-02/064123
- US-A- 6 106 930
- US-A1- 2004 219 109
- US-B2- 6 596 298

## Description

### Field of the Invention

This invention relates to edible films, which contain a unit dosage of an orally administered medication.

### Background of the Invention

For convenient non-intrusive administration of oral hygiene agents there exist products, which comprise rapidly dissolvable non-self-adhering polymer-based edible thin film vehicles which contain antimicrobial agents and/or essential oils. LISTERINE^{®}POCKETPAK^{®} brand oral care strip products, made by Pfizer, Inc. of Morris Plains, N.J., are perhaps the most notable examples of such oral care edible film products.

In the LISTERINE^{®}POCKETPAK^{®} products, for the convenience of a user, multiple micro-thin translucent film strips (about 25-50 individual film strips) are combined in an overlying relationship one to another (e.g., in a stack) and placed in a compact container having an openable/closable container door. Upon opening of the container door, by finger pressure applied to the upper most film strip, a user can slide, detach or otherwise extract the upper most film strip from its underlying film strip in the container since the strips are of a non-self-adhering polymer. The user then orally consumes the extracted film strip to obtain a freshening of the mouth. Nevertheless, not infrequently upon a user's attempt to extract a single film strip there occurs an inadvertent and unnoticed extraction of two or more of the micro-thin film strips.
US Patent 2004/219109 describes medicated edible products like chewing gums having prints on their surface for identification of the active agent and the dosage contained.

Since the introduction of the LISTERINE^{®}POCKETPAK^{®} film strip product, there have been some proposals in the art for inclusion of unit dose amounts of other pharmaceutically active agents into micro-thin edible film strips like that of the LISTERINE^{®}POCKETPAK^{®} product. These proposals, such as are found in U.S. Patent 6,596,298 and U.S. Patent 6,740,332, presuppose the extraction from a unit dosage film container of only one strip of rapidly dissolving medicated edible film.

However, administration of a unit dosage of medication via an edible film strip could lead to inadvertent and unnoticed extraction from a container of multiple medicated strips.

Hence, there is a need for unit dosage medicated edible film strips, which reduce possible overdoses when multiple unit dosage medicated film strips are inadvertently extracted from a container in which they are housed. There is also a need for unit dosage medicated film strips which allow a user to purposefully extract a predetermined number of unit dosage medicated film strips.

### Summary of the Invention

The term "mark, marked, or marking" as used herein means forming indicia on a film by ink or laser printing, marking, embossing, stamping, engraving, texturizing, blotching, or any other type of indicia forming technique.

One embodiment of the invention provides a stack of at least two layers of an edible film comprising overlying and underlying film layers; each edible film contains a unit dosage of medication; each film within said stack having a unique marking, shape, cut-out, wording, color, texture, or other indicia (hereinafter, collectively referred to as "indicia") such that when one or more adjacent and overlying film layer(s) are extracted from said stack the indicia of the adjacent and overlying film layer(s) alone or in combination with one or more underlying film layer(s) inform a user of whether an appropriate dosage has been extracted.

Another embodiment of the invention provides a unit medicinal dosage distribution device comprising: a container for storage of multiple layers of an edible film; each edible film contains a unit dosage of medication; each film within said storage container having a unique indicia marked on the film layers such that when one or more adjacent and overlying film layer(s) are extracted from said stack the indicia of the adjacent and overlying film layer(s) alone or in combination with one or more underlying film layer(s) inform a user of whether an appropriate dosage has been extracted.

The present invention also relates to methods of preparing a stack of at least two layers of a unit dosage of edible films, which method comprises the steps of providing a ribbon of medicated edible film; cutting said ribbon into, preferably individually sized, stand alone, edible film strips containing a unit dosage of medication; marking or cutting said edible film strips with a unique indicia, such that the indicia represents individual dosage segments of one or more films; and stacking the individual dosage segments such that the unique indicia of one dosage segment is aligned with and overlaps the unique indicia of adjacent dosage segments in alternating order.

The present invention also relates to methods of preparing a stack of at least two layers of a unit dosage of edible films, which method comprises the steps of providing, preferably individually sized, stand alone, edible film strips; marking or cutting said edible film strips with a unique indicia, such that the indicia represents individual dosage segments of one or more films; and stacking the individual dosage segments such that the indicia of one dosage segment is aligned with and overlaps the indicia of adjacent dosage segments in alternating order.

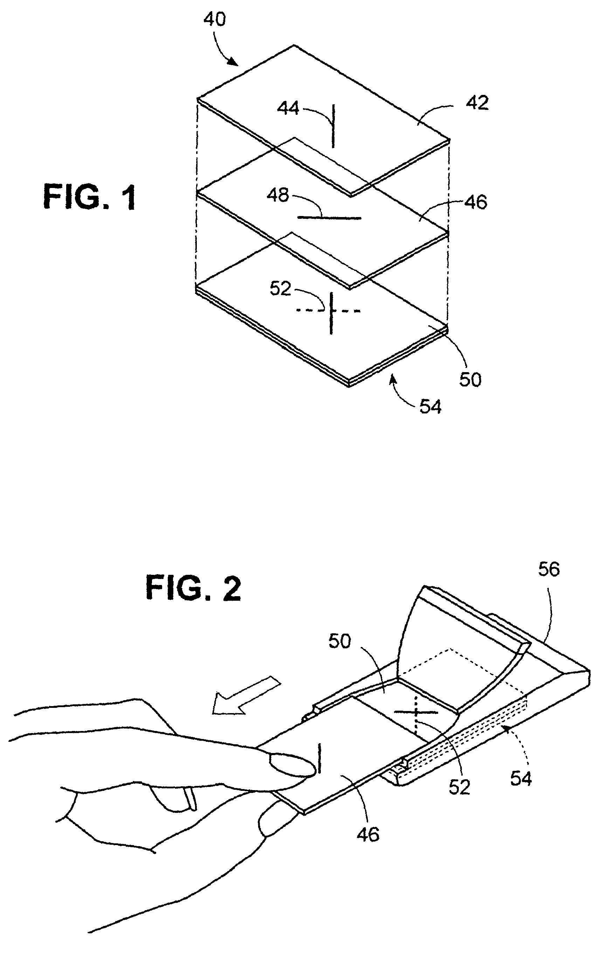

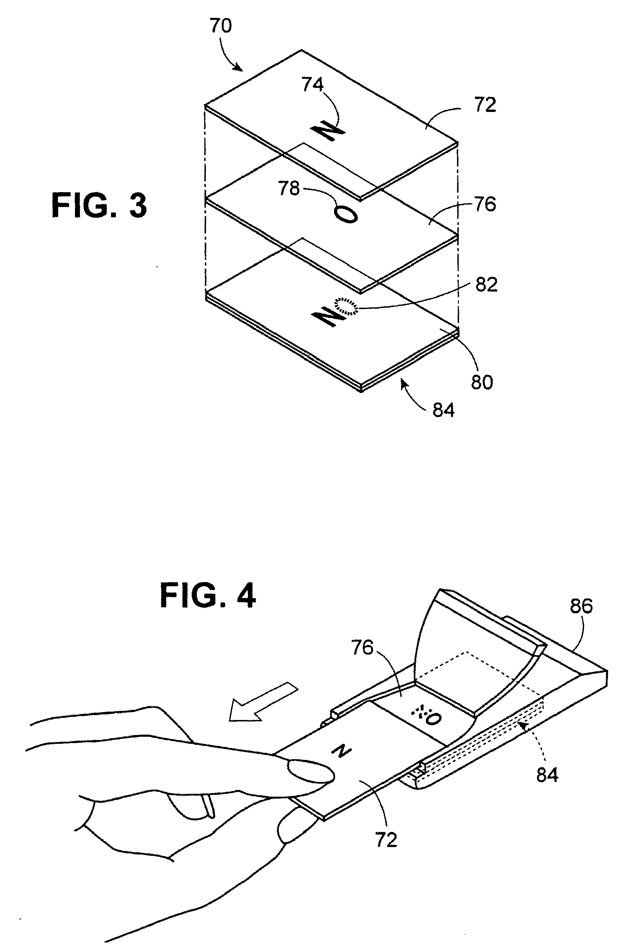

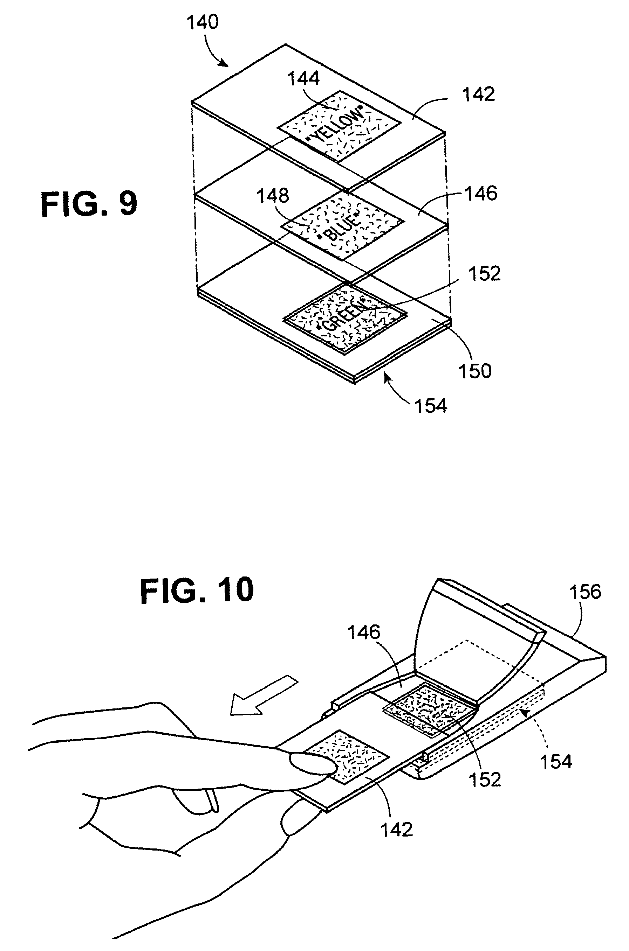

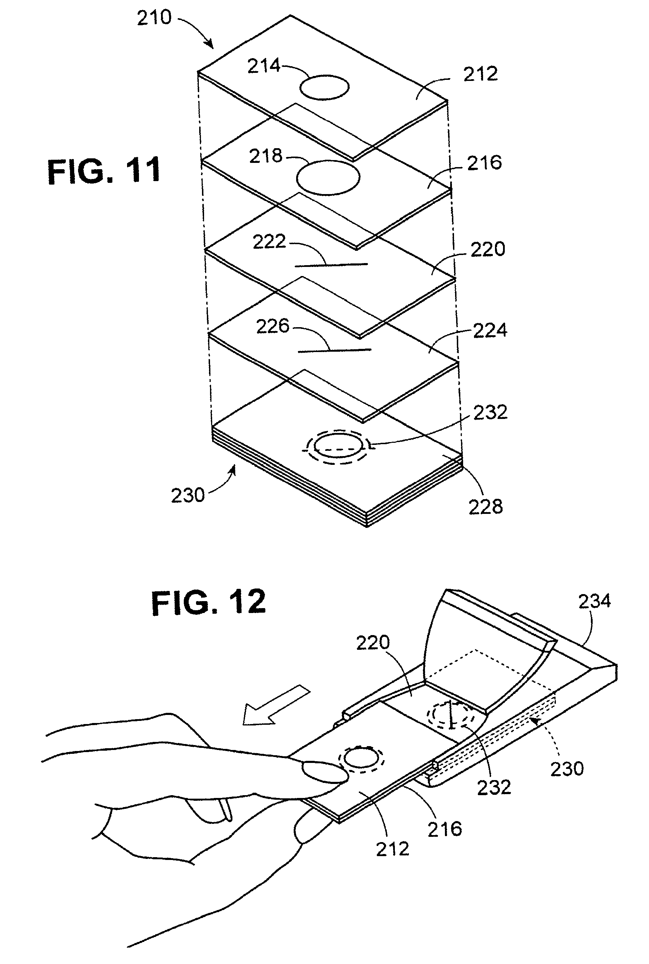

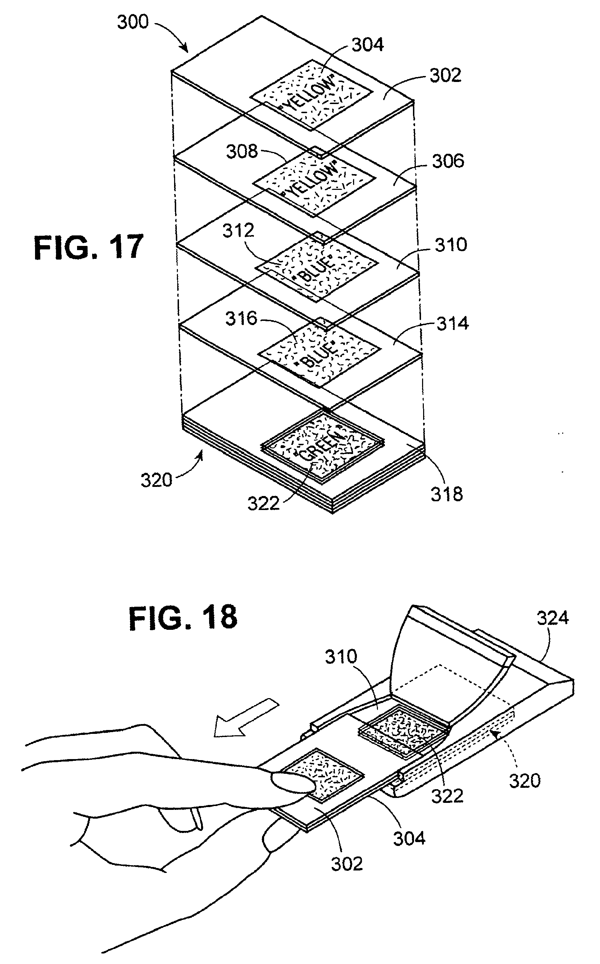

Where the film layers are translucent, a unique mark, shape, cut-out, word, color, or other indicia on one or more film layer(s) underlying one or more adjacent overlying film layer(s) can be discerned (seen) through the overlying film layer(s) and the individual film markings are viewable as a unitary mark indicating an overdose situation (See FIGS. 1 and 11). Other means of uniquely marking film strips may be used, such as alternating off-set lettering (See FIG 3). Additionally, alternate film strips may be uniquely and differently marked compared one to another by different coloring to indicate an overdose situation (See FIGS. 9 and 17). Moreover, one skilled in the art will readily perceive that any marking mechanism, including unique indicia can be used to effectively avoid an overdose of medicated edible film strips.

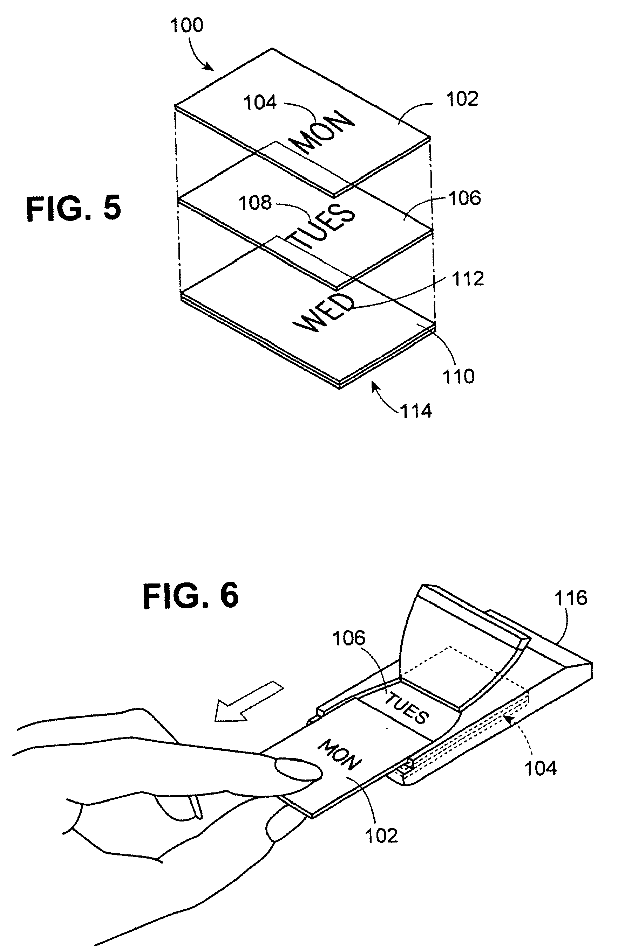

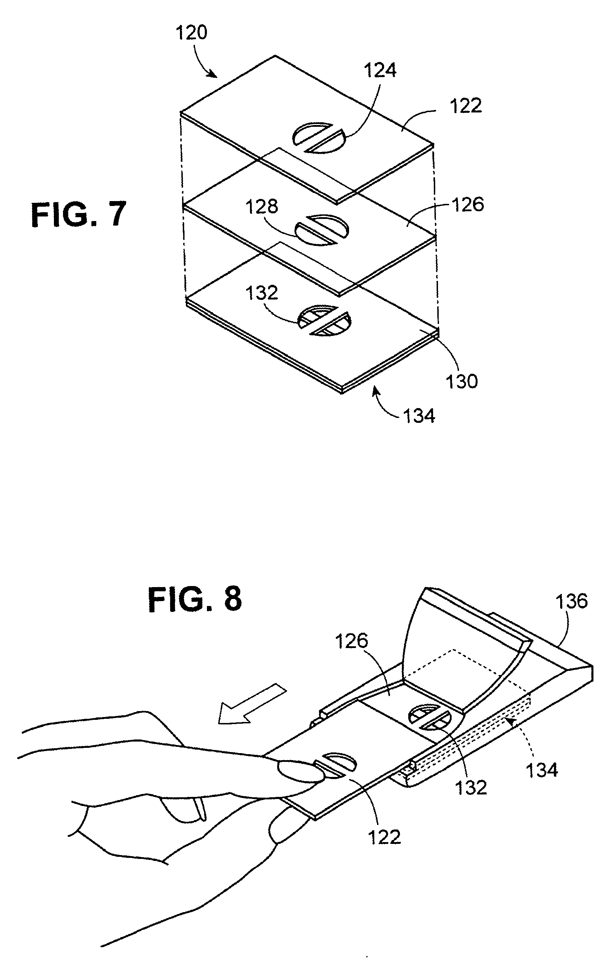

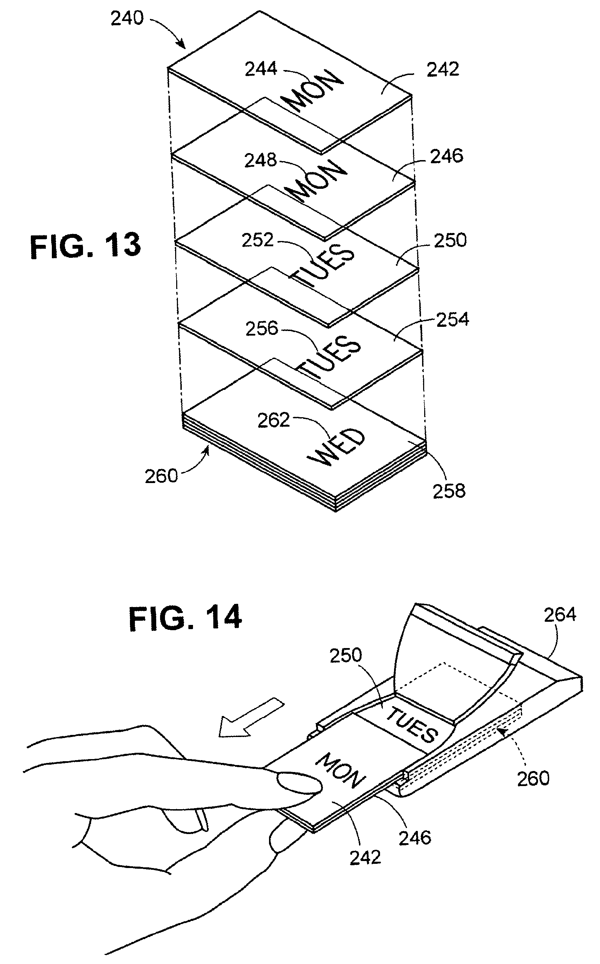

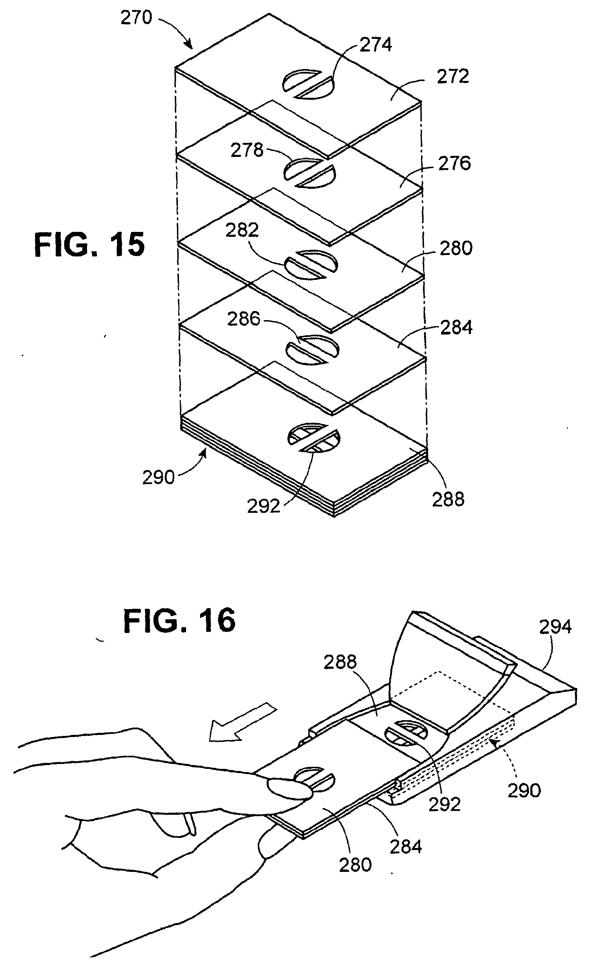

When the film layers are non-translucent, a mark such as a word or other indicia on one or more film layer(s) can indicate an overdose situation (See FIGS. 5 and 13). Additionally, when the film layers are non-translucent, a shape, cut-out, or other indicia on one or more film layer(s) can be discerned (seen) through one or more overlying film layer(s) to the one or more underlying film layer(s) such that individual film shapes or cut-out-type indicia in total or in combination are viewable as a unitary shape or cut-out indicating an overdose situation. (See FIGS. 7 and 15). Moreover, one skilled in the art will readily perceive that any marking mechanism, including unique indicia can be used to effectively avoid an overdose of medicated edible film strips.

### Brief Description of the Drawings

Figure 1 illustrates a stack of translucent medicated edible film strips, which form by overlay a universal "X" (NO) symbol.
Figure 2 illustrates a container, containing translucent medicated edible film strips, wherein a hand is removing a single medicated film dosage.
Figure 3 illustrates a stack of translucent medicated edible film strips, which form by overlay a universal "NO" symbol
Figure 4 illustrates a container, containing translucent medicated edible film strips, wherein a hand is removing a single medicated film dosage.
Figure 5 illustrates a stack of medicated edible film strips, which show a daily dose by a "day" marking (e.g., MON, TUES, WED).
Figure 6 illustrates a container, containing medicated edible film strips, wherein a hand is removing a single medicated film dosage.
Figure 7 illustrates a stack of medicated edible film strips, which contain shape cut-outs and form by overlay a "+" symbol.
Figure 8 illustrates a container, containing medicated edible film strips, wherein a hand is removing a single medicated film dosage.
Figure 9 illustrates a stack of translucent medicated edible film strips in different colors symbolized by various dot markings, which form by overlay a composite color designating an overdose condition.
Figure 10, illustrates a container, containing translucent medicated film strips, wherein a hand is removing a single medicated film dosage.
Figure 11 illustrates a stack of translucent medicated edible film strips where the dosage segment requires multiple film strips, which dosage segments form by overlay a universal " 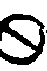 " (NO) symbol.
Figure 12 illustrates a container, containing translucent medicated edible film strips where the dosage segment requires multiple film strips, wherein a hand is removing from the container multiple strips for a predetermined dosage.
Figure 13 illustrates a stack of medicated edible film strips where the dosage segment requires multiple film strips, which dosage segments show a daily dose by a "day" marking (e.g., MON, TUES).
Figure 14 illustrates a container, containing translucent medicated edible film strips where the dosage segment requires multiple film strips, wherein a hand is removing from the container multiple strips for a predetermined dosage.
Figure 15 illustrates a stack of medicated edible film strips where the dosage segment requires multiple film strips, which dosage segments form by overlay a "+" symbol.
Figure 16 illustrates a container, containing translucent medicated edible film strips where the dosage segment requires multiple film strips, wherein a hand is removing from the container multiple strips for a predetermined dosage.
Figure 17 illustrates a stack of translucent medicated edible film strips where the dosage segment requires multiple film strips, which dosage segments form by overlay a composite color designating an overdose condition.
Figure 18 illustrates a container, containing translucent medicated edible film strips where the dosage segment requires multiple film strips, wherein a hand is removing from the container multiple strips for a predetermined dosage.

### Detailed Description of the Invention

The invention is a physiologically acceptable edible film that dissolves in a mouth of a user, which film carries a unit dose of a pharmaceutically active agent, and, which film is shaped or marked with a shape, cut-out, figure, color, hologram, mark, word, texture, or other indicia, which is unique compared to an adjacent film, which it overlies or underlies such that when more than the appropriate or predetermined number of film layers are inadvertently extracted from a container containing a stack of such film layers, the situation becomes apparent to the user, which deters one from a possible over dosage situation. The term "unique" as used herein means any shape, cut-out, figure, color, hologram, mark, word, texture, or other form(s) of indicia, placed on the film whether by blotching, coloring, cutting, embossing, engraving, marking, printing, shaping, stamping, and/or texturizing etc., that informs the consumer of the dosage parameters and/or characteristics of a particular film.

The physiologically acceptable edible film can be of any composition heretofore identified by the art, such as the films described by U.S. Patent 6,596,298 B2 and/or U.S. Patent 6,740,332 B2. The edible films of the present invention also encompass water soluble or erodible versions of such teeth whitening films as that described in U.S. Patent Application 20040086468 and U.S. Patent Nos. 6,419,906, 6,780,401, 6,045,811. As well as films formed by such hot melt processes as that described in US Patent No. 6,375,963. In certain embodiments, the film in its final form is translucent in comparison to an underlying film adjacent and compatible to the medicine it contains. These conditions are readily ascertainable for any film-medicament combination without undue experimentation by those of ordinary skill in the art. These edible films may be fast-dissolving or simply erodible (slow-dissolving) to provide sustained-release type preparations. To a film forming solution as described by U.S. Patent 6,596,298 B2 and/or U.S. Patent 6,740,332 B2, or other edible film forming solutions, a quantity of a medication is added and uniformly dispersed therein which when the film is cast there from and preferably cut to a user intended size (e.g., 1" x I") for packaging, and later consumption, will contain per a single film strip thereof a unit dosage of that medication. As will be discussed in further detail hereafter, the film is then shaped and/or marked, optionally using edible ink, either before it is cut or slit into ribbon form or after it is cut to ribbon form, to provide a unique marking to what will become individual film strips made there from and packaged in an overlying-underlying relationship one to another. That is, within a package of say 25-50 film strips or any number of multiple strips, each film strip in the stack will have a mark, which is unique to it in comparison to a film strip that overlies or underlies it in the stack.

The edible film includes at least one physiologically acceptable, pharmaceutically active agent. These agents should be compatible with the film. Suitable pharmaceutically active agents include, but are not limited to: antimicrobial agents, non-steroidal anti-inflammatory drugs, anti-tussives, decongestants, anti-histamines, expectorants, anti-diarrheals, H₂ -antagonists, proton pump inhibitors, nonselective CNS depressants, nonselective CNS stimulants, drugs that selectively modify CNS function, antiparkinsonism drugs, narcotic-analgesics, analgesic-antipyretics, psychopharmacological drugs, antianginal, antimigraine, and the like.

Examples of antimicrobial agents are triclosan, cetyl pyridium chloride, domiphen bromide, quaternary ammonium salts, zinc compounds, sanguinarine, fluorides, alexidine, octonidine, EDTA, and the like.

Examples of non-steroidal anti-inflammatory drugs are aspirin, acetaminophen, ibuprofen, ketoprofen, diflunisal, fenoprofen calcium, flurbiprofen sodium, naproxen, tolmetin sodium, indomethacin, celecoxib, valdecoxib, paracoxib and rofecoxib, analgesics LTD-4, LTB-4 and 5-LO inhibitors and the like.

Examples of anti-tussives are benzonatate, caramiphen edisylate, menthol, dextromethorphan hydrobromide, chlophedianol hydrochloride, and the like.

Examples of decongestants are pseudoephedrine hydrochloride, phenylepherine hydrochloride, phenylpropanolamine, pseudoephedrine sulfate, and the like.

Examples of anti-histamines are brompheniramine maleate, chlorpheniramine maleate, carbinoxamine maleate, clemastine fumarate, dexchlorpheniramine maleate, diphenhydramine hydrochloride, diphenylpyraline hydrochloride, azatadine maleate, diphenhydramine citrate, doxylamine succinate, promethazine hydrochloride, pyrilamine maleate, tripelennamine citrate, triprolidine hydrochloride, acrivastine, loratadine, desloratadine, brompheniramine, dexbrompheniramine, fexofenadine, cetirizine, montelukast sodium and the like.

Examples of expectorants are guaifenesin, ipecac, potassium iodide, terpin hydrate, and the like.

An example of an anti-diarrheals is loperamide, and the like.

Examples of H₂ -antagonists are famotidine, ranitidine, and the like.

Examples of proton pump inhibitors are omeprazole, lansoprazole, and the like.

Examples of general nonselective CNS depressants are aliphatic alcohols, barbiturates and the like.

Examples of general nonselective CNS stimulants are caffeine, nicotine, strychnine, picrotoxin, pentylenetetrazol and the like.

Examples of drugs that selectively modify CNS function are phenyhydantoin, phenobarbital, primidone, carbamazepine, ethosuximide, methsuximide, phensuximide, trimethadione, diazepam, benzodiazepines, phenacemide, pheneturide, acetazolamide, sulthiame, bromide, and the like.

Examples of antiparkinsonism drugs are levodopa, amantadine and the like.

Examples of narcotic-analgesics areas morphine, heroin, hydromorphone, metopon, oxymorphone, levorphanol, codeine, hydrocodone, xycodone, nalorphine, naloxone, naltrexone and the like.

Examples of analgesic-antipyretics are salycilates, phenylbutazone, indomethacin, phenacetin and the like.

Examples of psychopharmacological drugs are chlorpromazine, methotrimeprazine, haloperidol, clozapine, reserpine, imipramine, tranylcypromine, phenelzine, lithium and the like.

Examples of antianginal agents are limaprost, nitroglycerin, nifedipine, bepridil and the like.

Examples of antimigraine drugs are sumitriptan succinate, zolmitriptan, valproic acid eletriptan hydrobromide and the like.

Mixtures of any of the above-mentioned pharmaceuticals may also be used.

The amount of pharmaceutically active agent that can be used in the rapidly dissolving films is dependent upon the dose needed to provide an effective amount of the active agent. An "effective amount" is meant to be an amount of the active agent that is sufficient to at least reduce or relieve the condition, symptom, or disease being treated, but low enough to avoid any adverse side effects. In addition to the particular active agent, the effective amount of the pharmaceutically active agent may vary with the type and/or severity of the disease, symptom or condition, the age and physical condition of the patient being treated, the duration of treatment, the nature of concurrent therapy, the specific form (i.e., salt) of the pharmaceutically active agent employed, and the particular carrier from which the pharmaceutically active agent is applied. The amount of the pharmaceutically active agent in the formulation may be adjusted to deliver a predetermined dose of the active agent over a predetermined period of time, which may typically vary from 4 to 24 hours. Some examples of doses for specific medicaments that can be delivered per one strip of rapidly dissolving oral film are set forth in Table 1.

**TABLE 1**

| MEDICAMENT | DOSE |
|---|---|
| Chlorpheniramine Maleate | 4-12 mg. |
| Brompheniramine Maleate | 4 mg. |
| Dexchlorpheniramine | 2 mg. |
| Dexbrompheniramine | 2 mg. |
| Triprolidine Hydrochloride | 2.5 mg. |
| Cetirizine | 5-10 mg. |
| Acrivastine | 8 mg. |
| Azatadine Maleate | 1 mg. |
| Loratadine | 5-10 mg. |
| Phenylephrine Hydrochloride | 5-10 mg. |
| Dextromethorphan Hydrochloride | 10-30 mg. |
| Sildenafil | 25-100 mg. |
| Ketoprofen | 12.5-25 mg. |
| Sumatriptan Succinate | 35-70 mg. |
| Zolmitriptan | 2.5 mg. |
| Loperamide | 2 mg. |
| Famotidine | 5-10 mg. |
| Nicotine | 1-15 mg. |
| Diphenhydramine Hydrochloride | 12.5-25 mg. |
| Pseudoephedrine Hydrochloride | 15-60 mg. |
| Atorvastatin | 5-80 mg. |
| Valdecoxib | 5-20 mg. |
| Amlodipine besylate | 2.5-10 mg. |
| Rofecoxib | 5-25 mg. |
| Setraline hydrochloride | 10-100 mg. |
| Ziprasidone | 20-80 mg. |
| Eletriptan | 10-40 mg. |
| Nitroglycerin | 0.3-0.6 mg. |

The edible film may also include at least one nutritionally acceptable component such as vitamins, minerals, trace elements, fibers, and mixtures thereof. Examples of vitamins suitable for the film include Vitamin A, Vitamin D, Vitamin E, Vitamin K, Vitamin C, folic acid, thiamin, riboflavin, Vitamin B (6), Vitamin B (12), niacin, biotin and panthotenic acid in pharmaceutical or nutritionally acceptable form. Examples of mineral elements and trace elements suitable for the film include calcium, sodium, potassium, phosphorous, magnesium, manganese, copper, zinc, iron, selenium, chromium, and molybdenum in pharmaceutical or nutritionally acceptable form.

In preparing the film stacks of the present invention, a sheet of a medicated edible film is colored, cut, marked, and/or shaped with a pattern of marking or shaping such that when the sheet is cut to ribbons and the ribbons are stacked and the stacked ribbons are cut to the final product length, each film strip (or dosage segment of film strips) in the stack has a marking, shape, cut-out, wording, color, texture, or other indicia that is different from the film strip (or dosage segment of film strips), which is adjacent and underlying in the stack. The individual film strips (or dosage segment of film strips) are then stacked in an alternating pattern based on the different indicia, wherein the indicia is aligned and overlaps. Alternately, the sheet of a medicated edible film may be first cut to ribbons, then the individual ribbons may be put through a coloring, cutting, marking, and/or shaping station and marked with a pattern, optionally using edible ink, such that when the ribbons are stacked, each ribbon (or dosage segment of ribbon) so marked has a marking that is different than any ribbon (or dosage segment of ribbon), which is adjacent and underlying in the pile or group of ribbons. A pile or group of such ribbons may be laid out and a stack of film strips cut there-from wherein each film strip (or dosage segment of film strips) in the cut stack has a marking that is different than any film strip (or dosage segment of film strips), which is adjacent and underlying in the stack. In the case of translucent films, each marking or shape is aligned and configured such that the overlapping indicia form composite indicia. Also alternatively, a pile or group of such ribbon may be laid out and a stack of film strips cut there-from wherein each film strip (or dosage segment of film strips) in the cut stack has a shaping that is different than any film strip (or dosage segment of film strips), which is adjacent and underlying in the stack. The film strips are aligned and configured such that the shaping on the film strips overlap and form composite shapes.

Any pattern of alternate marking of adjacent film strips may be used. For example, a first dosage may include one or more film layer(s) printed with a forward slash sign ("/") and the next dosage underlying and alternate to the first dosage may include one or more film layer(s) printed with a back slash sign ("\") such that when more than the appropriate or predetermined number film layers are inadvertently extracted from their storage container, the user may observe the composite mark of "X" (FIG 1) indicating an overdose situation has occurred. Another pattern could be to mark one or more film layer(s) of one dosage offset to the left of its centerline with "ST" and the adjacent or underlying film layer(s) of another dosage offset to the right of its centerline with "OP" such that when more than the appropriate or predetermined number film layers are inadvertently extracted from their storage container, the user may observe the composite mark of "STOP" indicating an overdose situation has occurred. Other means of uniquely marking film strips may be used, such that the one or more overlying films may be printed with circles ("O") and the underlying film may be printed with a back slash sign "\" to indicate as an overdose situation by the composite viewable mark of " 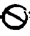 " (FIG 11). Other examples are to print one or more film layer(s) left off-center with "OVER" and its adjacent or underlying layer right off-center with "DOSE" for the composite mark of "OVER DOSE," or one or more film layer(s) left off-center with "DON'T" and its adjacent or underlying layer right off-center with "TAKE" for the composite mark of "DON'T TAKE." Another example would be to print one or more film layer(s) left off-center with "N" and its adjacent or underlying layer right off-center with "O" for the composite mark of "NO" (FIG 3).

Moreover, different shapes and/or cut-outs in a stack of multiple layers of edible film may be used. For example, a first dosage may include one or more film layer(s) containing horizontal ellipse (or ellipse-like) cut-outs and the next dosage underlying or alternate the first dosage, may include one or more film layer(s) containing vertical ellipse (or ellipse-like) cut-outs such that more than the appropriate or predetermined number film layers are inadvertently extracted from their storage container, the user may observe the composite cut-out of "+" (FIG 7) (FIG 15) indicating an overdose situation has occurred and the composite cut-out designating an overdose condition.

Alternatively, in the situation of translucent films, each translucent edible film containing a unit dosage of a medication may be dyed to a different color with an edible dye (i.e., yellow alternating with blue) such that one dosage set of one or more films is yellow with its alternate as blue. When more than the appropriate predetermined number of film layers are inadvertently extracted, the composite color of the extracted strips is green (FIG 9) (FIG 17), which designates an overdose condition. In the situation of non-translucent films, a first dosage may include one or more film layer(s) printed with a date (MON) and next dosage underlying or alternate to the first dosage may include one or more film layer(s) printed with another date (TUES) (FIG 5) (FIG.13) such that when more than the appropriate or predetermined number film layers are inadvertently extracted from their storage container, the user may observe the different dates to indicate that an overdose situation has occurred.

In certain embodiments, the marking uses edible inks. Many varieties of edible inks are known that may be used for printing of the medicated edible film. For example see U.S. Pat. No. 3,258,347 for "Edible Pharmaceutical Ink"; U.S. Pat. No. 2,948,626 for "Edible Pharmaceutical Ink and Process of Using Same"; U.S. Pat. No. 3,694,237 "Edible Ink"; U.S. Pat. No. 4,543,370 "Dry Edible Film Coating Composition, Method and Coating Form"; U.S. Pat. No. 5,006,362 for "Branding Pharmaceutical Dosage Forms, Food and Confectionery Products with Aqueous Ingestible Inks"; U.S. Pat. No. 5,453,122 for "Ink Composition"; and U.S. Pat. No. 6,623,553 for "Printing Process with Edible Inks".

Turning to FIG 1, a stack of translucent medicated edible film strips 40 is shown. A first strip 42 is marked with a forward slash ("/") mark 44. A second strip 46 is marked with a back slash mark ("\") 48. A third strip 50 is shown. A universal "X" (NO) symbol 52 is visible to a user viewing strip 50. The strips below strip 50 (i.e. remaining stack 54) together form the universal "X" (NO) symbol 52 by the overlay of the separate components provided on the individual strips in stack 54. An individual universal "X" (NO) symbol 52 is formed by the overlay or stacking of more than one strip.

Turning to FIG 2, a unit medicinal dosage distribution device 56 is shown, wherein a single translucent strip 46 is removed from a stack of strips 54. A universal "X" (NO) symbol 52 is visible to a user viewing strip 50. The strips below strip 50 (i.e. remaining stack 54) together form the universal "X" (NO) symbol 52 by the overlay of the separate components provided on the individual strips in stack 54.

Turning to FIG 3, a stack of translucent medicated edible film strips 70 is shown. A first strip 72 is marked with a left off-center "N" mark 74. A second strip 76 is marked with a right off-center "O" 78. A third strip 80 is shown. A universal "NO" symbol 82 is visible to a user viewing strip 80. The strips below strip 80 (i.e. remaining stack 84) together form the universal "NO" symbol 82 by the overlay of the separate components provided on the individual strips in stack 84. An individual universal "NO" symbol 82 is formed by the overlay or stacking of more than one strip.

Turning to FIG 4, a unit medicinal dosage distribution device 86 is shown, wherein a single translucent strip 72 is removed from a stack of strips 84. A universal "NO" symbol 82 is visible to a user viewing strip 76. The strips below strip 76 (i.e. remaining stack 84) together form the universal "NO" symbol 82 by the overlay of the separate components provided on the individual strips in stack 84.

Turning to FIG 5, a stack of non-translucent medicated edible film strips 100 is shown. A first strip 102 is marked with a day symbol "MON" mark 104. A second strip 106 is marked with a day symbol "TUES" mark 108. A third strip 110 is marked with a day symbol "WED" mark 112.

Turning to FIG 6, a unit medicinal dosage distribution device 116 is shown, wherein a single non-translucent strip 102 is removed from a stack of strips 114. Non-translucent strip 102 is marked by a day symbol "MON" mark. Non-translucent strip 106 is marked with a day symbol "TUES" mark.

Turning to FIG 7, a stack of medicated edible film strips 120 is shown. A first strip 122 is marked with a cut-out shape 124. A second strip 126 is marked with a cut-out shape 128. A third strip 130 is shown. A "+" symbol 132 is visible to a user viewing strip 130. The strips below strip 130 (i.e. remaining stack 134) together form the "+" symbol 132 by the overlay of the separate components provided on the individual strips in stack 134. An individual "+" symbol 132 is formed by the overlay or stacking of more than one strip.

Turning to FIG 8, a unit medicinal dosage distribution device 136 is shown, wherein a single strip 122 is removed from a stack of strips 134. A "+" symbol 132 is visible to a user viewing strip 126. The strips below strip 126 (i.e. remaining stack 134) together form the "+" symbol 132 by the overlay of the separate components provided on the individual strips in stack 134.

Turning to FIG 9, a stack of translucent medicated edible film strips 140 is shown. A first strip 142 symbolizes the color yellow by various dot markings 144. A second strip 146 symbolizes the color blue by various dot markings 148. A third strip 150 is shown. The color green is symbolized by various dot markings 152 and is visible to a user viewing strip 150. The strips below strip 150 (i.e. remaining stack 154) together form the composite color green by the overlay of the separate components provided on the individual strips in stack 154. The composite color green is formed by the overlay or stacking of mare than one strip.

Turning to FIG 10, a medicinal dosage distribution device 156 is shown, wherein a single translucent strip 142 is removed from a stack of strips 154. A composite green color symbolized by various dot markings 152 is visible to a user viewing strip 146. The strips below strip 154 (i.e. remaining stack 154) together form the composite green color 152 by the overlay of the separate components provided on the individual strips in stack 154.

Turning to FIG 11, a stack of translucent medicated edible film strips 10 is shown. A first strip 12 is marked with a small circle 14. A second strip 16 is marked with a relatively larger circle 18. A third strip 20 is marked with a back slash ("\") mark 22. A duplicate fourth strip 24 is marked with a back slash ("\") mark 26. A fifth strip 28 is shown. A universal " 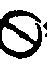 " (NO) symbol 32 is visible to a user viewing strip 28. The strips below strip 28 (i.e. remaining stack 30) together form the universal " 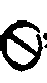 " (NO) symbol 32 by the overlay of the separate components provided on the individual strips in stack 30. An individual universal " 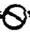 " (NO) symbol 32 is formed by the overlay or stacking of more than two strips.

Turning to FIG 12, a unit medicinal dosage distribution device 34 is shown, wherein multiple translucent strips 12 and 16 are removed from a stack of strips 30. A universal " 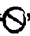 " (NO) symbol 32 is visible to a user when viewing strip 20 in the distribution device 34. The strips below strip 20 (i.e. remaining stack 30) together form the universal " 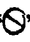 " (NO) symbol 32 by the overlay or stacking of the separate components provided on more than two strips in stack 30.

Turning to FIG 13, a stack of non-translucent medicated edible film strips 40 is shown. A first strip 42 is marked with a day symbol "MON" mark 44. A second strip 46 is marked with a day symbol "MON" mark 48. A third strip 50 is marked with a day symbol "TUES" mark 52. A fourth strip 54 is marked with a day symbol "TUES" mark 56. A fifth strip 58 is marked with a day symbol "WED" mark 62.

Turning to FIG 14, a unit medicinal dosage distribution device 64 is shown, wherein multiple non-translucent strips 42 and 46 are removed from a stack of strips 60. Non-translucent strips 42 and 46 are marked with a day symbol "MON" mark 44 and 48. Non-translucent strip 50 in the distribution device 64 is marked with a day symbol "TUES" mark.

Turning to FIG. 15, a stack of medicated edible film strips 70 is shown. A first strip 72 is marked with a cut-out shape 74. A duplicate second strip 76 is marked with a cut-out shape 78. A third strip 80 is marked with a cut-out shape 82. A duplicate fourth strip 84 is marked with a cut-out shape 86. A "+" symbol 92 is visible to a user viewing strip 88. The strips below strip 88 (i.e. remaining stack 90) together form the "+" symbol 92 by the overlay of the separate components provided on the individual strips in stack 90. An individual "+" symbol 92 is formed by the overlay or stacking of more than two strips.

Turning to FIG. 16, a unit medicinal dosage distribution device 94 is shown, wherein multiple strips 80 and 84 are removed from a stack of strips 90. A "+" symbol 92 is visible to a user viewing strip 88. The strips below strip 88 (i.e. remaining stack 90) together form the "+" symbol 92 by the overlay or stacking of the separate components provided on more than two strips in stack 90.

Turning to FIG 17, a stack of translucent medicated edible film strips 100 is shown. A first strip 102 symbolizes the color yellow by various dot markings 104. A duplicate second strip 106 symbolizes the color yellow by various dot marking 108. A third strip 110 symbolizes the color blue by various dot markings 112. A duplicate strip 114 symbolizes the color blue by various dot markings 116. A fifth strip 118 is shown. The color green is symbolized by various dot markings 122 and is visible to a user viewing strip 118. The strips below strip 118 (i.e. remaining stack 120) together form the composite color green 122 by overlay of the separate components provided on the individual strips in stack 120. The composite color green is formed by the overlay or stacking of more than two strips.

Turning to FIG 18, a unit medicinal dosage distribution device 124 is shown, wherein multiple translucent strips 102 and 104 are removed from a stack of strips 120. A composite color green symbolized by various dot markings 122 is visible to a user viewing strip 110. The strips below strip 120 (i.e. remaining stack 120) together form the composite color green 122 by the overlay or stacking of the separate components provided on more than two strips in stack 120.

## Claims

1. A stack of at least two layers of an edible film comprising overlying and underlying film layers; each edible film contains a unit dosage of medication; each film within said stack having a unique marking, shape, cut-out, wording, color, texture, or other indicia such that when one or more adjacent and overlying film layer(s) are extracted from said stack the unique marking, shape, cut-out, wording, color, texture, or other indicia of the adjacent and overlying film layer(s) alone or in combination with one or more underlying film layer(s) inform a user of whether an appropriate dosage has been extracted.

2. The stack of claim 1, wherein the mark of one or more adjacent and overlying layer(s) is / and the mark of its underlying layer is \ and the composite marking designating an overdose condition is X.

3. The stack of claim 1, wherein the mark of one or more adjacent and overlying layer(s) is N and the mark of its underlying layer is O and the composite marking designating an overdose condition is NO.

4. The stack of claim 1, wherein the mark of one or more adjacent and overlying layer(s) is ST and the mark of its underlying layer is OP and the composite marking designating an overdose condition is STOP.

5. The stack of claim 1, wherein the color of one or more adjacent and overlying layer(s) is yellow and the color of the underlying layer is blue and the composite color designating an overdose condition is green.

6. The stack of claim 1, wherein the film of one or more adjacent and overlying layer(s) contain a horizontal ellipse cut-out and the underlying layer contains a vertical ellipse cut-out and the composite shape designating an overdose condition is +.

7. The stack of claim 1, wherein the medication is selected from the group consisting of antimicrobial agents, non-steroidal anti-inflammatory drugs, anti-tussives, decongestants, anti-histamines, expectorants, anti-diarrheals, H₂-antagonists, proton pump inhibitors, nonselective CNS depressants, nonselective CNS stimulants, drugs that selectively modify CNS function, antiparkinsonism drugs, narcotic-analgesics, analgesic-antipyretics, psychopharmacological drugs, antianginal, antimigraine, nutritionally acceptable components, and mixtures thereof.

8. A stack of at least two layers of a translucent edible film comprising overlying and underlying film layers; each edible film contains a unit dosage of medication; each film within said stack having a unique marking, shape, cut-out, wording, color, or other indicia such that when one or more adjacent and overlying translucent film layer(s) are extracted from said stack the unique marking, shape, cut-out, wording, color, or other indicia of the adjacent and overlying translucent film layer(s) in combination with one or more underlying film layer(s) are viewable as a composite marking, shape, cut-out, wording, color, or other indicia to inform a user of whether an appropriate dosage has been extracted.

9. The stack of claim 8, wherein the mark of one or more adjacent and overlying layer(s) is / and the mark of its underlying layer is \ and the composite marking designating an overdose condition is X.

10. The stack of claim 8, wherein the mark of one or more adjacent and overlying layer(s) is N and the mark of its underlying layer is O and the composite marking designating an overdose condition is NO.

11. The stack of claim 8, wherein the mark of one or more adjacent and overlying layer(s) is ST and the mark of its underlying layer is OP and the composite marking designating an overdose condition is STOP.

12. The stack of claim 8, wherein the color of one or more adjacent and overlying layer(s) is yellow and the color of the underlying layer is blue and the composite color designating an overdose condition is green.

13. The stack of claim 8, wherein the film of one or more adjacent and overlying layer(s) contain a horizontal ellipse cut-out and the underlying layer contains a vertical ellipse cut-out and the composite shape designating an overdose condition is +.

14. A unit medicinal dosage distribution device comprising: a container for storage of multiple layers of an edible film; each edible film contains a unit dosage of medication; each film within said storage container having a unique marking, shape, cut-out, wording, color, or other indicia marked on the film layers such that when one or more adjacent and overlying film layer(s) are extracted from said stack the unique marking, shape, cut-out, wording, color, texture, or other indicia of the adjacent and overlying film layer(s) alone or in combination with one or more underlying film layer(s) inform a user of whether an appropriate dosage has been extracted.

15. A method of preparing a stack of at least two layers of a unit dosage of edible films, which method comprises the steps of:
a) providing a ribbon of medicated edible film;
b) cutting said ribbon into edible film strips containing a unit dosage of medication;
c) marking or cutting said edible film strips with a unique marking, shape, cut-out, wording, color, texture, or other indicia, such that the unique marking, shape, cut-out, wording, color, texture, or other indicia represents individual dosage segments of one or more films; and
d) stacking the individual dosage segments such that the unique marking, shape, cut-out, wording, color, texture, or other indicia of one dosage segment is aligned with and overlaps the unique marking, shape, cut-out, wording, color, texture, or other indicia of adjacent dosage segments in alternating order.

## Patentansprüche

1. Ein Stapel aus wenigstens zwei Schichten aus einem essbaren Film, der obenliegende und untenliegende Filmschichten umfasst; jeder essbare Film enthält eine Medikamentendosiseinheit; wobei jeder Film im Stapel eine einzigartige Markierung, Form, Ausschnitt, Beschriftung, Farbe, Textur oder andere Anzeige aufweist, sodass, wenn eine oder mehrere benachbarte und obenliegende Filmschicht(en) aus dem Stapel herausgezogen werden, die einzigartige Markierung, Form, Ausschnitt, Beschriftung, Farbe, Textur oder andere Anzeige der benachbarten und obenliegenden Filmschicht(en) allein oder in Kombination mit einer oder mehreren untenliegenden Filmschicht(en) einen Benutzer darüber informiert, ob eine richtige Dosierung herausgezogen worden ist.

2. Der Stapel nach Anspruch 1, wobei die Markierung einer oder mehrerer benachbarter und obenliegender Schicht(en) / ist und die Markierung ihrer untenliegenden Schicht \ ist und die zusammengesetzte Markierung, die eine Überdosis anzeigt, X ist.

3. Der Stapel nach Anspruch 1, wobei die Markierung einer oder mehrerer benachbarter und obenliegenden Schicht(en) N ist und die Markierung ihrer untenliegenden Schicht O ist und die zusammengesetzte Markierung, die eine Überdosis anzeigt, NO ist.

4. Der Stapel nach Anspruch 1, wobei die Markierung einer oder mehrerer benachbarter und obenliegender Schicht(en) ST ist und die Markierung ihrer untenliegenden Schicht OP ist und die zusammengesetzte Markierung, die eine Überdosis anzeigt, STOP ist.

5. Der Stapel nach Anspruch 1, wobei die Farbe einer oder mehrerer benachbarter und obenliegenden Schicht(en) gelb ist und die Farbe der untenliegenden Schicht blau ist und die zusammengesetzte Farbe, die eine Überdosis anzeigt, grün ist.

6. Der Stapel nach Anspruch 1, wobei der Film einer oder mehrerer benachbarter und obenliegenden Schicht(en) einen horizontalen Ellipsenausschnitt enthält und die untenliegende Schicht einen vertikalen Ellipsenausschnitt enthält und die zusammengesetzte Form, die eine Überdosis anzeigt, + ist.

7. Der Stapel nach Anspruch 1, **dadurch gekennzeichnet, dass** das Medikament ausgewählt ist aus der Gruppe, bestehend aus antimikrobiellen Mitteln, nicht-steroiden entzündungshemmenden Arzneistoffen, Hustenmitteln, Entstauungsmitteln, Antihistaminika, Expektorantien, Antidiarrhoika, H₂-Antagonisten, Protonenpumpeninhibitoren, nicht-selektiven ZNS-Sedativa, nicht-selektiven ZNS-Stimulantien, Arzneistoffen, die ZNS-Funktion selektiv modifizieren, Antiparkinsonmitteln, Narkoanalgetika, Analgetikantipyretika, Psychopharmaka, Antianginamittel, Antimigränemittel, emährungsmäßig verträglichen Komponenten und Mischungen davon.

8. Ein Stapel aus wenigstens zwei Schichten aus einem durchscheinenden essbaren Films, der obenliegende und untenliegende Filmschichten umfasst; jeder essbare Film enthält eine Medikamentendosiseinheit; wobei jeder Film im Stapel eine einzigartige Markierung, Form, Ausschnitt, Beschriftung, Farbe oder andere Anzeige aufweist, sodass, wenn eine oder mehrere benachbarte und obenliegende durchscheinende Filmschicht(en) aus dem Stapel herausgezogen werden, die einzigartige Markierung, Form, Ausschnitt, Beschriftung, Farbe oder andere Anzeige der benachbarten und obenliegenden durchscheinenden Filmschicht(en) in Kombination mit einer oder mehreren untenliegenden Filmschicht(en) als eine zusammengesetzte Markierung, Form, Ausschnitt, Beschriftung, Farbe oder andere Anzeige sichtbar ist, um einen Benutzer darüber zu informieren, ob eine richtige Dosierung herausgezogen worden ist.

9. Der Stapel nach Anspruch 8, worin die Markierung einer oder mehrerer benachbarter und obenliegender Schicht(en) / ist und die Markierung ihrer untenliegenden Schicht \ ist und die zusammengesetzte Markierung, die eine Überdosis anzeigt, X ist.

10. Der Stapel nach Anspruch 8, wobei die Markierung einer oder mehrerer benachbarter und obenliegenden Schicht(en) N ist und die Markierung ihrer untenliegenden Schicht O ist und die zusammengesetzte Markierung, die eine Überdosis anzeigt, NO ist.

11. Der Stapel nach Anspruch 8, wobei die Markierung einer oder mehrerer benachbarter und obenliegender Schicht(en) ST ist und die Markierung ihrer untenliegenden Schicht OP ist und die zusammengesetzte Markierung, die eine Überdosis anzeigt, STOP ist.

12. Der Stapel nach Anspruch 8, wobei die Farbe einer oder mehrerer benachbarter und obenliegenden Schicht(en) gelb ist und die Farbe der untenliegenden Schicht blau ist und die zusammengesetzte Farbe, die eine Überdosis anzeigt, grün ist.

13. Der Stapel nach Anspruch 8, wobei der Film einer oder mehrerer benachbarter und obenliegenden Schicht(en) einen horizontalen Ellipsenausschnitt enthält und die untenliegende Schicht einen vertikalen Ellipsenausschnitt enthält und die zusammengesetzte Form, die eine Überdosis anzeigt, + ist.

14. Eine Medikamentendosiseinheitsverteilungsvorrichtung, welche umfasst: einen Behälter zur Aufbewahrung mehrerer Schichten aus einem essbaren Film; jeder essbare Film enthält eine Medikamentendosiseinheit; wobei jeder Film im Aufbewahrungsbehälter eine einzigartige Markierung, Form, Ausschnitt, Beschriftung, Farbe oder andere Anzeige aufweist, die auf den Filmschichten angebracht ist, sodass, wenn eine oder mehrere benachbarte und obenliegende Filmschicht(en) aus dem Stapel herausgezogen werden, die einzigartige Markierung, Form, Ausschnitt, Beschriftung, Farbe, Textur oder andere Anzeige der benachbarten und obenliegenden Filmschicht(en) allein oder in Kombination mit einer oder mehreren untenliegenden Filmschicht(en) einen Benutzer darüber informiert, ob eine richtige Dosierung herausgezogen worden ist.

15. Ein Verfahren zur Herstellung eines Stapels aus wenigstens zwei Schichten einer Dosiseinheit aus essbaren Filmen, wobei das Verfahren die Schritte umfasst:
a) Bereitstellen eines Bandes aus essbarem Medikamentenfilm;
b) Schneiden des Bandes in essbare Filmstreifen, die eine Medikamentendosiseinheit enthalten;
c) Markieren oder Schneiden der essbaren Filmstreifen mit einer einzigartigen Markierung, Form, Ausschnitt, Beschriftung, Farbe, Textur oder anderen Anzeige, sodass die einzigartige Markierung, Form, Ausschnitt, Beschriftung, Farbe, Textur oder andere Anzeige individuelle Dosissegmente aus einem oder mehreren Filmen darstellt; und
d) Stapeln der individuellen Dosissegmente, sodass die einzigartige Markierung, Form, Ausschnitt, Beschriftung, Farbe, Textur oder andere Anzeige eines Dosissegmentes mit der einzigartigen Markierung, Form, Ausschnitt, Beschriftung, Farbe, Textur oder anderen Anzeige benachbarter Dosissegmente in alternierender Reihenfolge fluchtet und diese überlappt.

## Revendications

1. Pile d'au moins deux couches d'un film comestible comprenant des couches de film surjacentes et sous-jacentes ; chaque film comestible contient un dosage unitaire de médicament ; chaque film dans ladite pile ayant un marquage, une forme, une découpe, un libellé, une couleur, une texture uniques ou d'autres indices de sorte que quand une ou plusieurs couches de film adjacentes et surjacentes sont extraites de ladite pile, le marquage, la forme, la découpe, le libellé, la couleur, la texture uniques ou d'autres indices de la (des) couche(s) de film adjacente(s) et surjacente(s) seule ou en combinaison avec une ou plusieurs couches de film sous-jacentes informent l'utilisateur si un dosage approprié a été extrait.

2. Pile selon la revendication 1, dans lequel la marque d'une ou de plusieurs couches adjacentes et surjacentes est /, et la marque de sa couche sous-jacente est \, et le marquage composite désignant une condition de surdosage est X.

3. Pile selon la revendication 1 dans lequel la marque d'une ou plusieurs couches adjacentes et surjacentes est N et la marque de sa couche sous-jacente est O et le marquage composite désignant une condition de surdosage est NO.

4. Pile selon la revendication 1, dans laquelle la marque des une ou plusieurs couches adjacentes et surjacentes est ST et la marque de sa couche sous-jacente est OP et le marquage composite désignant une condition de surdosage est STOP.

5. Pile selon la revendication 1, dans lequel la couleur des une ou plusieurs couches adjacentes et surjacentes est le jaune et la couleur de la couche sous-jacente est le bleu et la couleur composite désignant une condition de surdosage est le vert.

6. Pile selon la revendication 1, dans laquelle le film d'une ou de plusieurs couches adjacentes et surjacentes contient une découpe elliptique horizontale et la couche sous-jacente contient une découpe elliptique verticale et la forme composite désignant une condition de surdosage est +.

7. Pile selon la revendication 1, dans lequel le médicament est choisi dans le groupe comprenant les agents antimicrobiens, les médicaments anti-inflammatoires non stéroïdiens, les antitussifs, les décongestionnants, les antihistaminiques, les expectorants, les antidiarrhéiques, les antagonistes H₂, les inhibiteurs de pompe à protons, les dépressifs non sélectifs du système nerveux central, les stimulants du système nerveux central non sélectifs, les médicaments qui modifient sélectivement la fonction du système nerveux central, les médicaments contre la maladie de Parkinson, les narco-analgésiques, les analgésiques antipyrétiques, les médicaments psychopharmaceutiques, les anti-angineux, les anti-migraineux, les composants nutritivement acceptables, et leurs mélanges.

8. Pile d'au moins deux couches d'un film comestible translucide comprenant des couches de film surjacentes et sous-jacentes ; chaque film comestible contient un dosage unitaire de médicament ; chaque film dans ladite pile ayant un marquage, une forme, une découpe, un libellé, une couleur uniques ou d'autres indices de sorte que lorsqu'une ou plusieurs couches de film translucide adjacentes et surjacentes sont extraites de ladite pile, le marquage, la forme, la découpe, le libellé, la couleur uniques ou d'autres indices des couches de film translucide adjacentes et surjacentes en combinaison avec une ou plusieurs couches de film sous-jacentes peuvent être observés comme un marquage, une forme, une découpe, un libellé, une couleur, ou d'autres indices composites pour informer un utilisateur si un dosage approprié a été extrait.

9. Pile selon la revendication 8, dans lequel la marque d'une ou de plusieurs couches adjacentes et surjacentes est /, et la marque de sa couche sous-jacente est \, et le marquage composite désignant une condition de surdosage est X.

10. Pile selon la revendication 8, dans lequel la marque des une ou plusieurs couches adjacentes et surjacentes est N et la marque de sa couche sous-jacente est O et le marquage composite désignant une condition de surdosage est NO.

11. Pile selon la revendication 8, dans lequel la marque des une ou plusieurs couches adjacentes et surjacentes est ST et la marque de sa couche sous-jacente est OP et le marquage composite désignant une condition de surdosage est STOP.

12. Pile selon la revendication 8, dans laquelle la couleur des une ou plusieurs couches adjacentes et surjacentes est le jaune et la couleur de la couche sous-jacente est le bleu et la couleur composite désignant une condition de surdosage est le vert.

13. Pile selon la revendication 8, dans laquelle le film des unes ou plusieurs couches adjacentes et surjacentes contient une découpe elliptique horizontale et la couche sous-jacente contient une découpe elliptique verticale et la forme composite désignant une condition de surdosage est +.

14. Dispositif de distribution de dosage médicinal unitaire comprenant : un récipient pour stocker plusieurs couches d'un film comestible ; chaque film comestible contient un dosage unitaire de médicament ; chaque film à l'intérieur dudit récipient de stockage ayant un marquage, une forme, une découpe, un libellé, une couleur uniques ou d'autres indices marqués sur les couches de film de sorte que lorsqu'une ou plusieurs couches de film adjacentes et surjacentes sont extraites de ladite pile, le marquage, la forme, la découpe, le libellé, la couleur, la texture uniques ou d'autres indices de la (des) couche(s) de film adjacente(s) et surjacente(s) seule ou en combinaison avec une ou plusieurs couches de film sous-jacentes informent un utilisateur si un dosage approprié a été extrait.

15. Procédé pour préparer une pile d'au moins deux couches d'un dosage unitaire de films alimentaires, lequel procédé comprend les étapes consistant à :
a) prévoir un ruban de film comestible médicamenteux ;
b) couper ledit ruban en bandes de film comestible contenant un dosage unitaire de médicament ;
c) marquer ou couper lesdites bandes de film comestible avec un marquage, une forme, une découpe, un libellé, une couleur, une texture uniques ou d'autres indices de sorte que le marquage, la forme, la découpe, le libellé, la couleur, la texture uniques ou d'autres indices présentent des segments de dosage individuels d'un ou plusieurs films ; et
d) empiler les segments de dosage individuels de sorte que le marquage, la forme, la découpe, le libellé, la couleur, la texture uniques ou d'autres indices d'un segment de dosage sont alignés avec et chevauchent le marquage, la forme, la découpe, le libellé, la couleur, la texture uniques ou d'autres indices des segments de dosage adjacents dans un ordre alterné.
